# EUROPEAN PATENT APPLICATION

(11) **EP 2 388 008 A1**
(43) Date of publication of application: **23.11.2011**
(21) Application number: 10382126.0
(22) Date of filing: 14.05.2010
(51) Int. Cl.: A61K 31/7036, A61K 9/12

(54) **Stable, preservative-free, aqueous formulation for the administration by aerosolization comprising tobramycin**

(71) Applicant: Combino Pharm, S.L., 08970 Sant Joan Despi, Barcelona (ES)
(72) Inventor: Puigvert Colomer, Marina, E-08014, Barcelona (ES); Lloret Pérez, Sergio, E-08030, Barcelona (ES)
(74) Representative: ABG Patentes, S.L.

(57) **Abstract**

A stable, preservative-free aqueous formulation for the administration by aerosolization comprising from about 40 mg/ml to about 80 mg/ml of tobramycin, from about 0.10% to about 0.40% w/v of sodium chloride and has a pH value between about 3.0 and about 5.5

## Description

The present invention relates to a stable, preservative-free, aqueous tobramycin formulation for the administration by aerosolization, which formulation has an improved chemical stability as well as a process for its preparation.

### BACKGROUND OF THE INVENTION

Tobramycin is a water-soluble aminoglycoside, broad-spectrum antibiotic obtained from cultures of Streptomyces tenebrarius. It is effective against gram-negative bacteria, especially the pseudomonas species. Tobramycin acts by binding to a site on the bacterial ribosome, causing the genetic code to be misread. Similar to other aminoglycosides, it works by inhibiting bacterial protein synthesis through irreversible binding to the 30S ribosomal subunit of susceptible bacteria. Tobramycin is actively transported into the bacterial cell where it binds to receptors present on the 30S ribosomal subunit. This binding interferes with messenger RNA (mRNA). As a result, abnormal, non-functional proteins are formed due to misreading of the bacterial DNA. Eventually, susceptible bacteria die because of the lack of functional proteins. One aspect essential to aminoglycoside lethality is the need to achieve intracellular concentrations in excess of extracellular ones. Anaerobic bacteria are not susceptible to aminoglycosides due, at least in part, to a lack of an active transport mechanism for aminoglycoside uptake. Tobramycin as a medicament can be employed systematically and locally for the treatment of serious infections. Tobramycin can be employed as an injection given intramuscularly or intravenously, indicated for the treatment of very serious bacterial infections caused by susceptible strains of the designated microorganisms, as well as an ophthalmic solution - a topical antibiotic indicated in the treatment of external infections of the eye and its adnexa caused by susceptible bacteria. Tobramycin can also be administrated as a solution or dispersion for inhalation, where tobramycin is inhaled into the lungs to treat lung infections in patients with cystic fibrosis.

It is well known that tobramycin is chemically unstable. Generally, aminoglycosides and particularly tobramycin intravenous and ophtalmic formulations contain phenol or other preservatives to maintain potency and to minimize the production of degradation products that may increase impurities levels in the pharmaceutical product. However in case of tobramycin administered by aerosolization, phenol and other preservatives, when aerolized may induce bronchospasm and unwanted occurrence in patients with lung disease, and thereby inhibit its therapeutic safety and effectiveness.

US 5508269 tries to solve this apparent problem describing a preparation without any preservatives, with about 200 mg to 400 mg of aminoglycoside dissolved in a volume of about 5 ml. As an isotonising adjuvant it contains sodium chloride in an amount of about 0.225% which value is suitable to deliver aminoglycoside (tobramycin) to endobronchial space and the pH of the preparation is adjusted to a pH between about 5.5 and about 6.5 which is considered to be safe. However, this formulation was described to be stable only during 6 months at 25°C, which means that commercially available product should be stored under special conditions such as cooling during its shelf life.

Another liquid formulation of aminoglycoside (tobramycin) administered by aerosolization is disclosed in EP 1273292 B1. More precisely EP 1273292 B1 describes an aerosol formulation consisting of 75 mg/ml of tobramycin dissolved in 0.45% w/v sodium chloride aqueous solution wherein the pH is comprised between 4.0 and 5.5 and the osmolarity ranges between 250 and 450 mOsm/l providing significant improvement over existing prior art. However even this formulation was described to be stable only during 9 months at 25 °C.

Therefore there is still a need for preservative-free aqueous aminoglycoside formulation for administration by aerosolization, as the aerosolization is a suitable, well-tolerated and efficient method to deliver active ingredient into the endobronchial space for treating bacteria infections associated to pulmonary disease. In particular, there is still a need to provide the preservative-free aqueous tobramycin formulation for administration by aerosolization, where existing problems related to the poor chemical stability of tobramycin in formulations for administration by aerosollization are solved, providing a stable product guaranteeing maximal tolerance and efficacy, and which product could be stored at room temperature.

### SUMMARY OF THE INVENTION

The present invention provides a stable, preservative-free aqueous tobramycin formulation, which formulation is for the administration by aerosolization and is characterized in that it comprises about 40 mg/ml to about 80 mg/ml of tobramycin , about 0.10 to about 0.40 % w/v of sodium chloride and has a pH value between about 3.0 and about 5.5. The term tobramycin according to the present application includes tobramycin base or one of its salts. The salts of tobramycin include all pharmaceutically acceptable salts, such as, for example, sulfate, hydrochloride, nitrate or phosphate salt of tobramycin .

Under pathological conditions involving mucus hypersecretion and diminished mucociliary function such as cystic fibrosis, bacterial organisms may colonize the airways and produce infection, a process that leads to pulmonary tissue damage. Antibiotics are administered to reverse the lung infection by killing the responsible bacteria. In general, short-term therapy involves intravenous administration of high doses of antibiotics. In the present invention aerosol administration has been suggested for the delivery of tobramycin as an alternative to the other routes of antibiotic administration because, in general, high blood levels of antibiotics are necessary to attain adequate concentrations in the sputum. Aerosolized tobramycin has the advantage of delivering high concentrations directly to the site of infection and reducing the systemic concentrations of antibiotic. In this invention tobramycin has been formulated for the administration by aerosolization (for inhalation), as high systemic concentrations necessary to be effective against pulmonary P.aeruginosa can cause nephrotoxity and ototoxity.

An important advantage of the formulation of the present invention is its chemical stability. In general, stability of pharmaceutical product is a tendency of an active ingredient to resist change or decomposition due to internal reaction, or due to the action of heat, humidity, light, pressure, air etc. It also means the length of time that the drug retains its properties without loss of potency, and it usually is referred to as shelf life. Shelf testing is the best method to determine the performance of an aerosol product. The ingredients that make up the inhaler may be individually stable and compatible, but once they are blended together in the formulation, compatibility issues can arise.

Very often preservatives are used in pharmaceutical preparations to maintain potency and to minimize the production of degradation products that may increase impurities levels in the composition. However in case of tobramycin which is administred by aerosolization, phenol and other known preservatives, when aerolized induce bronchospasm and other unwanted occurrence in patients with lung disease and thereby inhibit its therapeutic safety and effectiveness. Therefore it is an object of present invention to provide an aqueous formulation of tobramycin for the administration by aerosolization which has improved stability. The term "stable" as used throughout the specification and claims, is meant to refer to the pharmaceutical product of the present invention which is chemically stable at room temperature during at least 12 months, preferably during 3 years, and for which the impurities profile and level fulfil at least with the requirements of ICH guidelines, European Pharmacopoeia, US Pharmacopoeia. This provides its therapeutic effectiveness and safety and also allows easy transport, handling and storing of the product without any special conditions such as cooling. The storage conditions reflect the potential shipping, warehousing, and marketing conditions. The term "room temperature" defines a temperature within the range of from about 10°C to about 38°C, preferably from about 20°C to about 25°C.

In accordance with the present invention it was surprisingly found that only a specific relation between the tobramycin concentration, sodium chloride concentration and the pH value can provide a pharmaceutical product with improved stability. The formulation according to the invention contains from about 40mg/ml to about 80mg/ml of tobramycin which is the minimal efficacious amount of tobramycin to supress the bacterial infections in endobronchail space. Lower doses are not sufficient to supress the bacterium and to treat the infection. Higher concentration preparation has also a disadvantages, as the concentration of tobramycin is higher than it's clinically needed, and also inadmissible precipitation of active ingredient was observed during formulation development of tobramycin product.

The osmolarity and pH grade are important in liquid, drug-product formulation for administration by aerosolization especially since it involves drug solubility, activity, absorption, stability, sorption and patient comfort. Tonicity and pH, though typically regarded as formulation issues, were investigated during preformulation for tobramycin to ensure selection of appropriate values of these parameters for development. For example, acidic (pH<3) hypertonic and hypotonic aerosols have been demonstrated to induce bronchoconstriction in asthmatic subjects. It is important to identify the pH together with sodium chloride concentration to ensure optimum product's stability, which should be monitored using a stability-indicating assay, following stress storage as a function of elevated temperature. In addition,conditions that promote degradation of active ingredient, such as extreme pH values or photolysis (ultraviolet and visible light), storage temperature, all merit consideration. As a guide to formulation development, studies were undertaken to evaluate the contribution of candidate excipients on compound stability. This is particularly important in the development of formulations of tobramycin. Tests performed during pharmaceutical development of tobramycin preparation, clearly indicated that at pH greater than 7.0 degradation of tobramycin occured, which was reflected in yellowing of the formulation indicating the presence of chromophore degradation product. The pH between about 3.0 and about 5.5 linked with sodium chloride value of about 0.10 to about 0.40% weight per volume (w/v), was found to be optimal in terms of storage. In the extended stability studies complete stability was found at room temperature during 12 months, preferably during 3 years. The pH of the formulation of the invention can be adjusted by using acidifying and alkalizing agents. Acidifying agents such as, for example, glacial acetic acid, acetic acid, citric acid, fumaric acid, hydrochloric acid, lactic acid, malic acid, nitric acid, phosphoric acid, propionic acid, sodium phosphate, sulfuric acid, tartaric acid, may be used in a formulation to lower the pH and alkalizing agents such as, for example, ammonia solution, ammonium carbonate, diethanolamine, monoethanolamine, potassium hydroxide, sodium bicarbonate, sodium borate, sodium carbonate, sodium hydroxide, sodium phosphate dibasic, trolamine, may be used to increase the pH. Many times a composition of a formulation may already have the desirable pH or may contain one of these agents to achieve a desirable pH. In all cases, before finishing up preparing a liquid formulation, the pH must be checked and adjusted if needed.

In a prefered embodiment of the invention, tobramycin formulation for administration by aerosolization comprises preferably from about 0.20% to about 0.35% w/v, more preferably from about 0.22% to 0.23%, even more preferably about 0.225% w/v of sodium chloride which value is linked with a preferred pH of the product assuring the best chemical stability, and therefore the best product's property.

In a preferred embodiment of the present invention the preferred pH value of the composition is comprised between about 4.0 and about 5.5.

The osmolarity of the tobramycin formulation was studied precisely in order to prevent bronchoconstruction and subsequent adverse reaction during pulmonary delivery of drugs. According to literature it has been recommended that whenever possible formulations for administration by aerosolization should be formulated as isotonic solutions. Recent studies of the present invention surprisingly show that tobramycin formulation containing about 0.10 to about 0.40% (w/v), preferably about 0.20 to about 0.35% (w/v), more preferebly from about 0.22% to about 0.23% (w/v) and even more preferably about 0.225% (w/v) of sodium chloride is within the safe value of aerosols administered to cystic fibrosis patients. This is surprising because this concentration of sodium chloride in a formulation having a pH value between about 3.0 and about 5.5, preferably between about 4.0 and about 5.5 results in a liquid formulation of tobramycin which is slightly hypotonic. Therefore, the preservative-free aqueous formulation of the invention preferably has an osmolarity value between about 110 and about 250 mOsmol/kg. This value of osmolarity permits better absorption of tobramycin and the lysis of bacterial cells is more efficient than with other known tobramycin products. The combination of the pH value between about 3.0 and about 5.5 and osmolarity in the range of about 110 to about 250 mOsm/kg gives fast first therapy results and the formulation is more efficiently nebulized. It has to be noted that in this specific range of osmolarity of about 110 to about 250 mOsm/kg the risk of paradoxical bronchoconstriction is completely prevented, and that the droplet-size distribution of an aerosol is adequate and complies with requirement for the development of the pharmaceutical product for administration by aerosolization.

In a prefered embodiment of the invention, the stable preservative-free tobramycin formulation for administration by aerosolization has an osmolarity value between about 130 and about 200 mOsm/kg, even more preferably between about 150 and about 180 mOsm/kg.

The stable, preservative-free aqueous formulation of the invention can be for example in the form of a solution or dispersion. But in a preferred embodiment, the preservative-free aqueous formulation of the invention is in the form of a solution. Precisely the product of the invention is preferably in the form of a solution for administration by aerosolization.

Stable, preservative-free aqueous formulation of the invention, can be administered by aerosolization pulmonarily or nasally preferably using a nebulizer device. Nebulizers (air-jet or ultrasonic devices) are used for acute care of nonambulatory patients. Continuously operating nebulizers are devices that convert liquids into aerosols by high pressure gases, ultrasonic vibration or other methods. They allow the dose to be inhaled at an appropriate rate and particle size which ensures deposition of the preparation in the lungs. Solutions of drug candidates in nebulized aerosol droplet form are frequently employed especially if solid particles are shown to be unstable upon storage. The solutions may be used with variety of different air and ultrasonic nebulizers. Air-jet nebulizers for atomization are considered portable because of the availability of small compressed air pumps. Ultrasonic nebulizers have the advantage of being more portable because they generally do not require a source of compressed air. Doses administered by nebulization are much larger than doses in MDis and the liquid reservoir is limited in size, resulting in short, single duration therapy. Nebulizers provide very small droplets and high mass output. In a prefered embodiment the aqueous formulation of the invention is administered by a jet, ultrasonic or piezoelectric nebulizer.

The stable, preservative-free aqueous formulation of the invention can be prepared by any known process in the art, suitable to manufacture formulation for administration by aerosolization. In particular the process of the invention comprises the steps of (i) mixing the tobramycin with at least sodium chloride in water to obtain a mixture, which is for example a solution or dispersion. The mixing step of the process should be carried out in an adequate container such as a tank or vessel under apropriate temperature conditions and speed of the propeler if used, assuring a safe and efficient process. After mixing, the process of the invention comprises the step of (ii) adjusting the pH if required to the desired value. The prepared mixture is then filtered through adequate filters to separate undissolved particles from the mixture if necessary. Further the process of the invention comprises the step of (iii) filling the mixture into the suitable container, which means transfer of the mixture into the final packaging material which can be of any form acceptable for pharmaceutical use such as glass type I, II or III container, or a plastic container. In the preferred embodiment, the product of the invention is filled into plastic containers, such as polyethylene or polypropylene plastic containers, by Blow Fill and Seal technology.

Deoxygenation (i.e. the removal of molecular oxygen for example by nitrogenation) may be conveniently used in some or all the steps of the manufacturing process. This provides an improvement of the process and better chemical stability of tobramycin product.

Throughout the description and claims the word "comprise" and variations of the word, are not intended to exclude other technical features, additives, components, or steps. The term "about" preceeding a value is meant to include non significant variations of the value given, preferably variations of not more than 5% of the given value, preferably the exact given value.

Additional objects, advantages and features of the invention will become apparent to those skilled in the art upon examination of the description. The following examples are provided by way of illustration, and they are not intended to be limiting of the present invention. Furthermore, the present invention covers all possible combinations of particular and preferred embodiments described herein.

### EXAMPLES

### Example 1.

### Preparation of the stable, preservative-free aqueous tobramycin formulation for the administration by aerosolization.

**Table. 01. Pharmaceutical composition of Batch 01 and Batch 02**

| Composition | [mg/vial 5ml] |
|---|---|
| Tobramycin | 300.000 mg |
| Sodium chloride | 11.250 mg |
| NaOH 0.5N solution or H₂SO₄ 3N and 0.01 N solution | q.s to pH 4.5 |
| Water for injection | q.s. to 5 mL |

**Table.02. Pharmaceutical composition of Batch 03**

| Composition | [mg/vial 5ml] |
|---|---|
| Tobramycin | 300.000 mg |
| Sodium chloride | 17.500 mg |
| NaOH 0.5N solution or H₂SO₄ 3N and 0.01 N solution | q.s to pH 4.5 |
| Water for injection | q.s. to 5 mL |

The stable, preservative-free aqueous tobramycin formulation for the administration by aerosolization was manufactured using the following procedure: Sodium chloride was dissolved in water for injection. The obtained solution was deoxygenated, and when a value of dissolved O₂ below 1 ppm was achieved, tobramycin was added until complete dissolution. The pH of the solution was determined, and adjusted with sulphuric acid and/or with sodium hydroxide solution to the value of about 3.0 to about 5.5. The solution was deoxygenated and when the value of dissolved O₂ below 1 ppm was achieved, the final solution was filtered through PVDF 0,45 µm and 0,22 µm filters, deoxygenated to obtain a value of dissolved O₂ less than 1 ppm, and filled into glass vials.

### Example 2.

### Stability results of the the stable, preservative-free aqueous tobramycin formulation for the administration by aerosolization.

Batch 01 of the pharmaceutical product comprising tobramycin produced according to Example 1. was subjected to stability tests. The stability studies were carried out with the following conditions as is defined in ICH Topic Q1A "Stability Testing of New Drug Substances and Products" (CPMP/ICH/2736/99).
<5°C & relative humidity not controlled - Condition A.
25 ± 2°C & 60% ± 5% relative humidity - Condition B.

**Table 3. Stability results of Batch 01**

| **Time (months)** / **Condition** | | **0** | **1 month** | | **6 months** | |
|---|---|---|---|---|---|---|
| **Test** / **Requirement** | | **N/A** | **A** | **B** | **A** | **B** |
| Appearance | *Clear solution free of particles* | Compl | Compl | Compl | Compl | Compl |
| pH | *Between 4.0 and 5.5* | 473 | 488 | 485 | 4 90 | 4 90 |
| Total Impurities | ≤ *1.5*% | 0 22% | 0 22% | 021% | 0 15% | 0 20% |
| Osmolarity | 110-250 mOsm/kg | 160 mOsm/kg | N/A | N/A | 162mOsm/kg | 162mOsm/kg |
| Chloride content | *34.5 - 42.35mmol*/*l* | 38 89 mmol/l | N/A | N/A | 35 59 mol/l | 39 29mol/l |

| | | | | | | |
|---|---|---|---|---|---|---|
| N/A- not applicable, ND- not detected. Compl.-complies | | | | | | |

All the tests were performed according to methods based on Eur.Ph. references and ICH guidelines requirements. It can be seen that the stability results of all tested parameters comply with the requirements. It should be noted that critical parameters such as related substances- total impurities, that were determined by HPLC, are well below the specified limits for conditions of 25 ± 2°C & 60% ± 5% relative humidity, ensuring high quality of the product comprising tobramycin.

### Example 3.

### Stability results of the stable, preservative-free aqueous tobramycin formulation for the administration by aerosolization.

Batch 02 of the pharmaceutical product comprising tobramycin produced according to example 1. was subjected to stability tests. The stability studies were carried out with the following conditions as is defined in ICH Topic Q1A "Stability Testing of New Drug Substances and Products" (CPMP/ICH/2736/99).
<5°C & relative humidity not controlled - Condition A.
25 ± 2°C & 60% ± 5% relative humidity - Condition B.

**Table 4. Stability results of Batch 02**

| **Time (months) Condition** | | **0** | **3** | | **6** | |
|---|---|---|---|---|---|---|
| **Test Requirement** | | **N/A** | **A** | **B** | **A** | **B** |
| Appearance | *Clear solution free of particles* | Compl | Compl | Compl | Compl | Compl |
| pH | *Between 4.0 and 5.5* | 477 | 499 | 522 | 4 76 | 473 |
| Total Impurities | ≤*1.5%* | 047% | 043% | 0 39% | 0 52% | 0 55% |
| Osmolarity | 110-250 mOsm/kg | 167 mOsm/kg | N/A | N/A | 168mOsm/kg | 176 mOsm/kg |

| | | | | | | |
|---|---|---|---|---|---|---|
| N/A- not applicable, ND- not detected. Compl.-complies | | | | | | |

All the tests were performed according to methods based on Eur.Ph. references and ICH guidelines requirements. It can be seen that the stability results of all tested parameters comply with the requirements. It should be noted that critical parameters such as related substances- total impurities that were determined by HPLC are well below the specified limits for conditions of 25 ± 2°C & 60% ± 5% relative humidity, ensuring high quality of the product comprising tobramycin.

## Claims

1. A Stable, preservative-free, aqueous formulation for the administration by aerosolization, **characterized in that** it comprises from about 40 mg/ml to about 80 mg/ml of tobramycin, from about 0.10% to about 0.40% w/v of sodium chloride and has a pH value between about 3.0 and about 5.5.

2. A stable, preservative-free, aqueous formulation according to claim 1, **characterized in that** it comprises preferably from about 0.20% to about 0.35% w/v, more preferably from about 0.22% to about 0.23% w/v and even more preferably about 0.225% w/v of sodium chloride.

3. A stable, preservative-free, aqueous formulation according to claim 1, **characterized in that** it has a pH value between about 4.0 and about 5.5.

4. A stable, preservative-free, aqueous formulation according to anyone claims 1 to 3, **characterized in that** it has an osmolarity value of about 110 to about 250 mOsmol/kg.

5. A stable, preservative-free, aqueous formulation according to claim 4, **characterized in that** it has an osmolarity value of about 130 to about 200mOsmol/kg, even more preferably of about 150 to about 180 mOsm/kg.

6. A stable, preservative-free, aqueous formulation according to anyone of claims 1 to 5, **characterized in that** it is in the form of a solution.

7. A stable, preservative-free, aqueous formulation according to anyone of claims 1 to 6, **characterized in that** it is suitable for administration by aerosolization pulmonarily or nasally using a nebulizer.

8. A stable, preservative-free, aqueous formulation according to claim 7, **characterized in that** it is suitable for administration by a jet, ultrasonic or piezoelectric nebulizer.

9. A process for the preparation of the stable, preservative-free aqueous formulation defined in anyone of claims 1 to 8, **characterized in that** it comprises the steps of: (i) mixing the tobramycin with sodium chloride in water (ii) adjusting the pH if required (iii) filtering the solution through adequate filters to separate undissolved particles from the mixture if necessary, and (iii) filling the mixture into a suitable container.
